(19) Europäisches Patentamt
European Patent Office
Office européen des brevets

(11) Veröffentlichungsnummer: **0 539 330 B1**

(12) **EUROPÄISCHE PATENTSCHRIFT**

(45) Veröffentlichungstag der Patentschrift: **17.05.95**

(51) Int. Cl.$^6$: **C07D 209/24**, C07D 209/18, C07C 69/96

(21) Anmeldenummer: **92810796.0**

(22) Anmeldetag: **16.10.92**

(54) **Verfahren zur Herstellung substituierter Indole.**

(30) Priorität: **25.10.91 CH 3136/91**

(43) Veröffentlichungstag der Anmeldung:
**28.04.93 Patentblatt 93/17**

(45) Bekanntmachung des Hinweises auf die Patenterteilung:
**17.05.95 Patentblatt 95/20**

(84) Benannte Vertragsstaaten:
**AT BE CH DE DK ES FR GB GR IE IT LI LU NL PT SE**

(56) Entgegenhaltungen:
**EP-A- 0 199 543**

**CHEMICAL ABSTRACTS, vol. 100, 1984, Columbus, Ohio, US; abstract no. 210430z, POZDNEV, V.F. 'N-(Cyclohexyloxycarbonyl)derivatives of amino acids' Seite 654 ;**

(73) Patentinhaber: **CIBA-GEIGY AG**
**Klybeckstrasse 141**
**CH-4002 Basel (CH)**

(72) Erfinder: **Gerspacher, Marc, Dr.**
**Ringstrasse 150**
**CH-4556 Aeschi (CH)**
Erfinder: **Sallmann, Alfred, Dr.**
**Joachimsackerstrasse 12**
**CH-4103 Bottmingen (CH)**

**EP 0 539 330 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neuartiges Verfahren zur Herstellung von Verbindungen der Formel I,

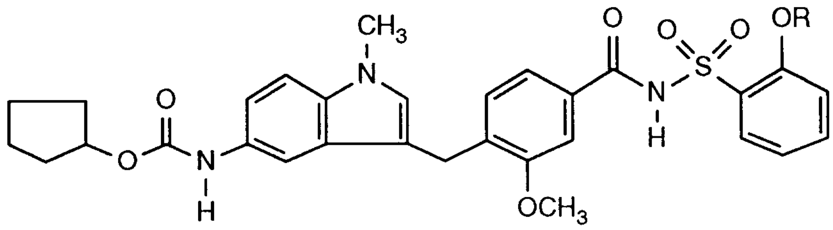

(I)

worin R geradkettiges $C_2$-$C_4$-Alk-1-en-1-yl bedeutet, oder Salzen davon, sowie ein neues Ausgangsmaterial und dessen Verwendung.

Die Verbindungen der Formel I, ihre pharmakologischen Eigenschaften und Verfahren zu ihrer Herstellung werden erstmals in EP-A-455 596 offenbart. In EP-A-199 543 und J. Med. Chem. 33 (1990) 1781-1790 sind Cyclopentyloxycarbonylamino-indole, die den Verbindungen der Formel I ähnlich sind, und Wege zu ihrer Herstellung beschrieben. Aus Houben-Weyl, Methoden der organischen Chemie, Band E4, Stuttgart 1983, Seiten 157-159 ist die Synthese einfacher Carbamidsäure-ester aus Dikohlensäure-diestern und aromatischen Aminen bekannt. Aus Chem. Abstract 100 (1984) 210430z ist die Acylierung von Aminosäuren mit Dicyclohexylpyrocarbonat bekannt.

Die Verbindungen I und ihre pharmazeutisch verwendbaren Salze weisen wertvolle pharmakologische Eigenschaften, insbesondere eine ausgeprägte antagonistische Wirkung gegenüber Leukotrienen, auf.

So hemmen sie z.B. in vitro in einem Konzentrationsbereich von etwa 0,001 bis etwa 1 µmol/l die durch Leukotrien $D_4$ ($LTD_4$) induzierte Kontraktion eines glatten Muskels. Diese als $LTD_4$-Antagonismus bezeichnete Wirkung kann experimentell z.B. verifiziert werden, indem in Segmenten, die dem Ileum eines 300 bis 400 g schweren Meerschweinchens entnommen und in einem Organbad in Tyrode-Lösung bei 38 °C und unter Begasung mit einem Gemisch aus 95 % Sauerstoff und 5 % Kohlendioxid bei einer Belastung von 1 g inkubiert wurden, mit synthetischem Leukotrien $D_4$ (in Form des Kaliumsalzes) Kontraktionen ausgelöst und diese isotonisch registriert werden. Das Ausmaß der Hemmung der Kontraktionen durch die Prüfsubstanz wird nach einer Vorinkubation von 2 Minuten in Form der $IC_{50}$ ermittelt, wobei als $IC_{50}$ diejenige Konzentration bezeichnet wird, bei welcher die Testkonträktionen um 50 % reduziert sind.

Die Verbindungen I und ihre pharmazeutisch verwendbaren Salze sind auch in vivo ausgezeichnet wirksam. So kann z. B. im Bronchokonstriktions-Standardtest am Meerschweinchen bei Verabreichung einer Aerosollösung, enthaltend von etwa 0,00001 bis etwa 1 Gewichtsprozent Prüfsubstanz, ein deutlicher $LTD_4$-antagonistischer Effekt beobachtet werden. In diesem Testmodell anästhetisiert man männliche, 400 bis 700 g schwere, Meerschweinchen intraperitoneal mit 1,4 g/kg Urethan und führt eine Polyethylenkanüle in die Vena jugularis ein. Eine zweite Polyethylenkanüle wird in die Trachea eingeführt. Mittels einer in die Speiseröhre eingeführten Kanüle, welche mit einem Statham-Druck-Transduktor verbunden ist, wird der Druck in der Speiseröhre aufgezeichnet. Das Tier wird in eine luftdicht verschließbare Kammer aus Plexiglas® gelegt, die mit einer Fleisch'schen Röhre Nr. 000 und einem Validyne-Transducer MP 45-1 verbunden ist. Mit dieser Anordnung wird der Flow gemessen. Nach der chirurgischen Präparierung der Versuchstiere wartet man eine gewisse Zeit, damit sich die pulmonalen Funktionen stabilisieren können. Die Prüfsubstanz wird anschließend gemäss dem nachfolgenden Protokoll verabreicht. Die Versuchstiere werden während einer Minute einer einprozentigen (Gewicht/Volumen) Aerosollösung der Prüfsubstanz oder destilliertem Wasser (zu Kontrollzwecken) ausgesetzt. Für alle Testverbindungen, welche durch Inhalation verabreicht werden, verwendet man ein Monaghan-Ultraschall-Sprühgerät (Modell 670), dessen Partikelgrösse sich zwischen 1 und 8 Mikron, mit einem Hauptanteil von 3 Mikron, bewegt. Wässrige Lösungen bzw. DMSO/Wasser-Gemische werden jeweils frisch hergestellt und mit einem on-stream drug vial in die Kammer des Sprühgeräts eingeführt. Der produzierte Sprühnebel wird den Versuchstieren über eine Glaskammer von 65 ml Inhalt, die mit einer Kanüle mit der Trachea verbunden wird, verabreicht. Nach Ablauf der Behandlungszeit wird mit einem zweiten Monaghan-Ultraschall-Sprühgerät (Modell 670) und über eine gleiche Glaskammer $LTD_4$ (0,3 µg/ml) während 2 Minuten verabreicht. Es wird die Abnahme der Compliance in der 3. Minute nach $LTD_4$-Applikation abgelesen, und zwar wird der Mittelwert von drei Tieren mit dem Mittelwert von drei Kontrolltieren verglichen und die prozentuale Hemmung der Compliance (% Hemmung) nach folgender Formel errechnet:

$$\% \text{ Hemmung} = 100 - \frac{(100 - \text{Compliance Präparat}) \cdot 100}{(100 - \text{Compliance Kontrolle})}$$

Werden unterschiedliche Wirkstoffkonzentrationen untersucht, so wird die prozentuale Hemmung für jede Konzentration aufgezeichnet, und zwar wird "log Konzentration" auf der Abszisse gegen "prozentuale Hemmung" auf der Ordinate aufgetragen. Die $IC_{50}$ wird dann durch lineare Regressionsanalyse ermittelt.

Die Verbindungen I und ihre pharmazeutisch verwendbaren Salze weisen weiterhin den spezifischen und therapeutisch sehr bedeutsamen Vorteil einer verhältnismäßig langen Wirkungsdauer auf.

Die Verbindungen I und ihre pharmazeutisch verwendbaren Salze können daher überall dort therapeutische Anwendung finden, wo die Wirkung der Leukotriene zu krankhaften Zuständen führt, und diese mildern oder beheben. Die Leukotriene spielen eine wichtige Rolle u. a. bei der Entstehung von allergischen und entzündlichen Prozessen. Demzufolge können die Verbindungen I und ihre pharmazeutisch verwendbaren Salze beispielsweise als Wirkstoffe in Antiallergika verwendet werden, welche z. B. zur Behandlung von allergischen Zuständen und Erkrankungen, wie insbesondere von Asthma, aber auch von Heufieber sowie von obstruktiven Lungenkrankheiten, einschließlich der zystischen Fibrose, eingesetzt werden.

Das Ziel intensiver Bemühungen besteht darin, ein kostengünstiges Verfahren mit besonders hohen Ausbeuten und besserer Handhabung sowie solche Verfahren zu entwickeln, bei denen das Durchlaufen instabiler Zwischenverbindungen weitgehend vermieden wird.

Das neuartige Verfahren ist dadurch gekennzeichnet, dass man

a) eine Verbindung der Formel IV,

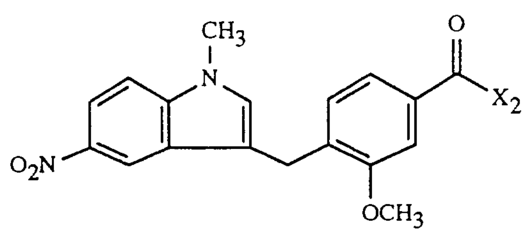

(IV)

worin $X_2$ für

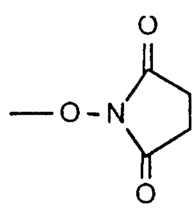

(IVa),

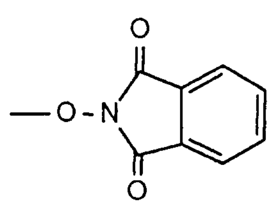

(IVb) oder

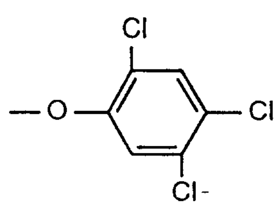

(IVc)

steht, in einem Eintopfverfahren, d.h. ohne Isolierung von Zwischenprodukten, reduziert und gleichzeitig mit der Verbindung der Formel V

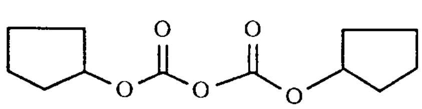

(V)

N-acyliert; und

b) eine so erhältliche Verbindung der Formel VIa

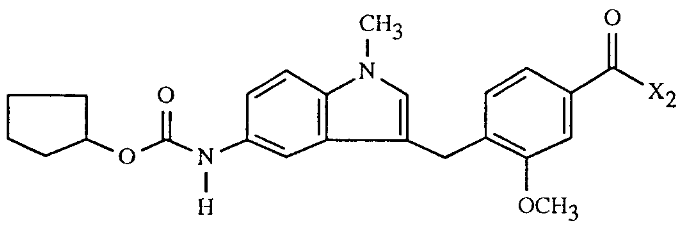

(VIa)

mit einer Verbindung der Formel VIb,

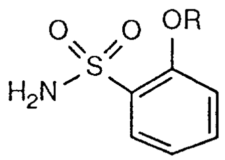

(VIb)

oder einen, Salz davon, umsetzt.

Insbesondere der Verfahrensschritt a) erweist sich überraschenderweise als besonders vorteilhaft, da dieser Reduktions- und N-Acylierungsschritt mit unerwartet hohen Ausbeuten verläuft. Dementsprechend bilden das Acylierungsreagenz der Formel V, Dicyclopentyl-dicarbonat, und seine Verwendung als Acylierungsreagenz für die Einführung einer Cyclopentyloxycarbonylgruppe bei der Verfahrensstufe a) ebenfalls einen Gegenstand der vorliegenden Erfindung.

Die Herstellung des Ausgangsmaterials der Formel V, Dicyclopentyl-dicarbonat, erfolgt durch Umsetzung von Halogenameisensäure-cyclopentylester, wie Chlorameisensäurecyclopentylester, in Gegenwart eines Amins, wie Trialkylamins, z.B. N,N-Dimethyl-N-octadecylamin,unter Verwendung einer anorganischen Base, wie Alkalimetallhydroxid, z.B. Natriumhydroxid. Dabei wird vorzugsweise in einem inerten, d.h. an der Reaktion nicht beteiligten Lösungsmittel gearbeitet.

Weiterhin weisen die Verbindungen der Formel IV und VIa vorteilhafte Kristallisationseigenschaften auf, die zusätzliche Reinigungsschritte, wie Chromatographie, entbehrlich machen.

Die als Ausgangsmaterial verwendeten Verbindungen der Formel IV werden dadurch hergestellt, dass man

a1) einen Ester der Formel II,

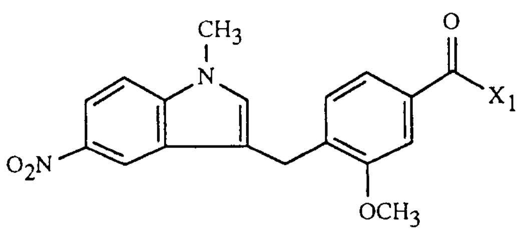

(II)

worin $X_1$ verethertes Hydroxy bedeutet, hydrolysiert; und

a2) in der so erhältlichen Carbonsäure der Formel III

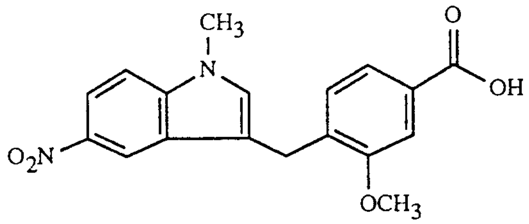

(III)

die Carboxygruppe durch Umsetzung mit einer Verbindung der Formel

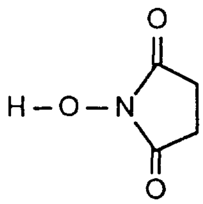

(IVd),

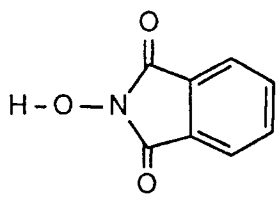

(IVe) oder

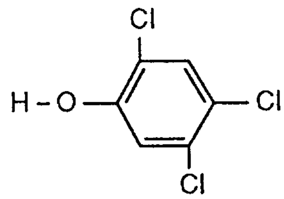

(IVf)

aktiviert.

Durch die Anwendung dieser neuartigen Verfahrensequenz [Verfahrensschritte a1), a2), a) und b)] werden überraschende Vorteile erzielt. So ist beispielsweise die Gesamtausbeute hoch, und die Isolierung von sich leicht zersetzenden Aminozwischenverbindungen, die aufwendige Reinigungsschritte erfordert, wird überflüssig gemacht.

Die Verbindungen der Formel IV sind ebenfalls zugänglich, indem man

1) in der Verbindung der Formel IIIa

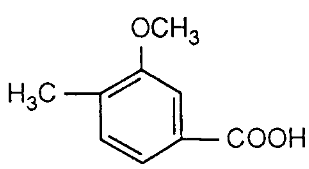

(IIIa)

die Carboxygruppe durch Umsetzung mit einer Verbindung der Formel IVd, IVe oder IVf (s. oben) aktiviert; und

2) eine so erhältliche Verbindung der Formel IIIb,

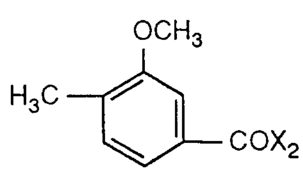

(IIIb)

worin $X_2$ für den Rest IVa, IVb oder IVc (s. oben) steht, halogeniert; und

3) eine so erhältliche Verbindung der Formel IIIc,

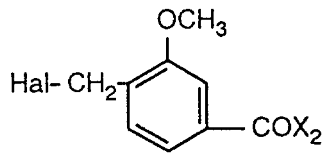

(IIIc)

worin Hal Halogen bedeutet und $X_2$ wie unter Formel IIIb definiert ist, mit der Verbindung der Formel IIId.

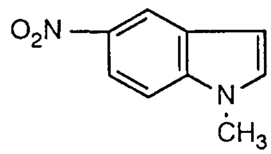

(IIId)

umsetzt.

Die Verbindungen der Formel I können im Rahmen der Erfindung, wenn der Rest R mehr als zwei Kohlenstoffatome besitzt und daher (E)- oder (Z)-Konfiguration aufweisen kann, in Form von Stereoisomeren, z. B. als reine Diastereomere oder Diastereomerengemische, vorliegen. Bevorzugt sind im Rahmen der Erfindung Verbindungen I, in welchen der Rest R die beispielsgemäss offenbarte Stereochemie aufweist.

Salze von Verbindungen der Formel I sind insbesondere pharmazeutisch verwendbare Salze, z. B. Säureadditionssalze, die beispielsweise mit starken anorganischen Säuren, wie Mineralsäuren, z. B. Schwefelsäure, einer Phosphorsäure oder einer Halogenwasserstoffsäure, mit starken organischen Carbonsäuren, wie Niederalkancarbonsäuren, z. B. Essigsäure, wie gegebenenfalls ungesättigten Dicarbonsäuren, z. B. Malon-, Malein- oder Fumarsäure, oder wie Hydroxycarbonsäuren, z. B. Wein- oder Zitronensäure, oder mit Sulfonsäuren, wie Niederalkan- oder gegebenenfalls substituierten Benzolsulfonsäuren, z. B. Methan- oder p-Toluolsulfonsäure, gebildet werden, oder Salze mit Basen, wie entsprechende Alkalimetall- oder Erdalkalimetallsalze, z. B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclischen Aminen, wie Mono-, Di- oder Triniederalkylaminen, wie Hydroxyniederalkylaminen, z. B. Mono-, Di- oder Trihydroxyniederalkylaminen, Hydroxyniederalkyl-niederalkylaminen oder Polyhydroxyniederalkylaminen. Cyclische Amine sind z. B. Morpholin, Thiomorpholin, Piperidin oder Pyrrolidin. Als Mononiederalkylamine kommen beispielsweise Ethyl- und t-Butylamin, als Diniederalkylamine beispielsweise Diethyl- und Diisopropylamin und als Triniederalkylamine beispielsweise Trimethyl- und Triethylamin in Betracht. Entsprechende Hydroxyniederalkylamine sind z. B. Mono-, Di- und Triethanolamin; Hydroxyniederalkyl-niederalkyl-amine sind z. B. N,N-Dimethylamino- und N,N-Diethylamino-ethanol; als Polyhydroxyniederalkylamin kommt z. B. Glucosamin in Frage. Umfasst sind ferner für pharmazeutische Verwendungen ungeeignete Salze, da diese beispielsweise für die Isolierung bzw. Reinigung von freien Verbindungen I sowie von deren pharmazeutisch verwendbaren Salzen verwendet werden können.

Vor- und nachstehend sind unter mit "Nieder" bezeichneten Resten und Verbindungen, sofern nicht abweichend definiert, solche zu verstehen, die bis und mit 7, vor allem bis und mit 4, C-Atome aufweisen.

Geradkettiges $C_2$-$C_4$-Alk-1-en-1-yl ist Vinyl, (Z)-Propen-1-yl, (E)-Propen-1-yl, (Z)-But-1-en-1-yl oder (E)-But-1-en-1-yl.

Bevorzugt sind im Rahmen der Erfindung Verbindungen der Formel I, worin R Vinyl, (Z)-Propen-1-yl oder (E)-Propen-1-yl bedeutet, und ihre Salze.

Besonders bevorzugt sind im Rahmen der Erfindung Verbindungen der Formel I, worin R Vinyl oder (Z)-Propen-1-yl bedeutet, und ihre Salze.

Ganz besonders bevorzugt sind im Rahmen der Erfindung die Verbindung der Formel I, worin R Vinyl bedeutet, und ihre Salze.

Die vor- und nachstehend beschriebenen Umsetzungen werden z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80 °C bis zur Siedetemperatur des Reaktionsmediums, vorzugsweise von etwa -20 ° bis etwa +150 °C, und, falls erforderlich, in einem geschlossenen Gefäss, unter Druck, in einer Inertgasatmosphare und/oder unter wasserfreien Bedingungen arbeitet Besonders vorteilhafte Reaktionsbedingungen

können den Beispielen entnommen werden.

Das vor- und nachstehend aufgeführte Ausgangsmaterial, das für die Herstellung der Verbindungen I und ihrer Salze verwendet wird, ist bekannt oder kann nach an sich bekannten Methoden, z.B. gemäss den nachstehend beschriebenen Verfahrensweisen, hergestellt werden.

Für Salze von vor- und nachstehend aufgeführten Ausgangsmaterialien gilt das vorstehend für Salze von Verbindungen I Gesagte in analoger Weise.

Verfahrensschritt a1):

Verethertes Hydroxy $X_1$ ist insbesondere Niederalkoxy, wie Methoxy.

Die Hydrolyse des Esters erfolgt in an sich bekannter Weise, beispielsweise z.B. in Gegenwart eines basischen Mittels oder eines sauren Mittels, wie einer Mineralsäure. Als Säuren kommen z.B. Schwefelsäure, eine Phosphorsäure oder eine Halogenwasserstoffsäure, eine starke organische Carbonsäure, wie gegebenenfalls, z.B. durch Halogen, substituierten $C_1$-$C_4$-Alkancarbonsäuren, z.B. Essigsäure, in Betracht. Als Basen kommen beispielsweise Alkalimetall-hydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxide, sowie carbocyclische Amine in Frage. Beispielhaft seien Lithium-, Natrium-hydroxid, -hydrid, -amid, Kalium-tert-butylat, Silber- oder Kalium-carbonat, Lithium-triphenylmethylid, -diisopropylamid, Kalium-3-(aminopropyl)-amid, -bis-(trimethylsilyl)-amid, Dimethylaminonaphthalin, Di- oder Triethylamin, oder Ethyl-diisopropylamin, N-Methyl-piperidin, Pyridin, Benzyltrimethyl-ammoniumhydroxid, 1,5-Diazabicyclo[4.3.0]non-5-en (DBN) sowie 1,8-Diaza-bicyclo[5.4.0]undec-7-en (DBU) genannt.

Vorzugsweise wird die Hydrolyse mit Hilfe von Lithiumhydroxid durchgeführt.

Als Lösungsmittel finden vor allem inerte, d.h. an der Umsetzung nicht beteiligte Lösungsmittel, oder Gemische davon, Anwendung, beispielsweise Ether, wie Tetrahydrofuran oder Dioxan, Alkohole, wie Niederalkanole, z.B. Methanol, Ethanol oder Isopropanol, oder Wasser.

Verfahrensschritt a2):

Bevorzugt wird der Rest $X_2$ =

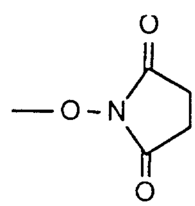 (IVa)

zur Aktivierung der Carboxygruppe eingeführt.

Die Aktivierung der Carboxygruppe in Verbindungen der Formel III erfolgt in Gegenwart eines Kondensationsmittels durch Umsetzung mit der entsprechenden Hydroxyverbindung, d.h. einer Verbindung der Formel IVd, IVe oder IVf (s. oben).

Als Kondensationsmittel kommt beispielsweise ein Carbodiimid, wie Diethyl- oder Dicyclohexyl-carbodiimid, oder, sofern man mit 2,4,5-Trichlorphenol umsetzt, zusätzlich eine Base, wie Diniederalkylamino-pyridin, z.B. Dimethylamino-pyridin, in Frage.

Die Umsetzung wird vorzugsweise in einen, Ether, wie Tetrahydrofuran oder Dioxan, halogenierte Kohlenwasserstoffe, wie Chloroform oder Tetrachlorkohlenstoff, ferner Ester, wie Ethylacetat, oder Amide, wie Dimethylformamid, durchgeführt.

Verfahrensschritt a):

Die Reduktion wird mit Hilfe eines die Reduktion der Nitrogruppe in Gegenwart einer aktivierten Carboxygruppe fördernden Hydrierungsnlittels durchgeführt. Als Reduktion kommt insbesondere die Hydrierung mit Wasserstoff in Gegenwart eines Hydrierungskatalysators, wie des Lindlarkatalysators oder Palladium auf Kohle, in Betracht.

Die N-Acylierung mit der Verbindung der Formel V wird ohne die Verwendung einer die N-Acylierung unterstützenden Base durchgeführt.

Als Lösungsmittel wird beispielsweise ein Ether, wie Tetrahydrofuran, Amide, wie Dimethylformamid, Ester, wie Ethylacetat, verwendet.

Verfahrensschritt b):

Die Umsetzung zum Sulfonamid wird beispielsweise in Gegenwart einer die Kondensation begünstigenden Base, durchgeführt. Als geeignete Base kommt beispielsweise 1,8-Diazabicyclo[5.4.0]undec-7-en (DBU), 1,5-Diazabicyclo[4.3.0]non-5-en (DBN), eine Alkalimetalalkoholat, wie Natriummethylat oder Kalium-tert-butylat, Benzyltrimethylammoniumhydroxid (Triton B®) oder ein stark basisches Amin, wie Diniederalkylaminopyridin, z.B. Dimethylamino-pyridin, in Frage. Bevorzugt wird als Base DBU eingesetzt.

Als Lösungsmittel wird beispielsweise ein Ether, wie Tetrahydrofuran, ein geeignetes Nitril, wie Acetonitril, ein Amid, wie Dimethylformamid, oder ein halogenierter Kohlenwasserstoff, wie Tetrachlorkohlenstoff, verwendet.

Verfahrensschritt 1):

Die Aktivierung der Carbonsäure erfolgt vorteilhaft in der in Verfahrensschritt a2) dargestellten Weise.

Verfahrensschritt 2):

Die Halogenierung wird in an sich bekannter Weise, beispielsweise unter Verwendung eines N-Halogen-succinimids, wie N-Brom-succinimid, durchgeführt.

Als Lösungsmittel wird beispielsweise ein halogenierter Kohlenwasserstoff, wie Tetrachlorkohlenstoff, verwendet.

Verfahrensschritt 3):

Die Alkylierung des Indolderivats IIId mit dem Benzylhalogenid der Formel IIIc wird in an sich bekannter Weise durchgeführt, z.B. in Gegenwart von Silbercarbonat und z.B. mit Toluol als Lösungsmittel.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes verwendet oder insbesondere unter den Reaktionsbedingungen bildet.

Die nachfolgenden Beispiele illustrieren die oben beschriebene Erfindung; sie sollen jedoch diese in ihrem Umfang in keiner Weise einschränken. Temperaturen sind in Grad Celsius angegeben.

Beispiel 1: [Verfahrensschritt b)] Zu einer Suspension von 10,0 g 4-[5-(Cyclopentyloxycarbonylamino)-1-methylindol-3-ylmethyl]-3-methoxy-benzoesäure-N-succinimidester und 4,1 g 2-Vinyloxy-benzolsulfonsäureamid in 150 ml Acetonitril setzt man unter Rühren und Einleiten von Argon bei Raumtemperatur 9,0 ml 1,8-Diazabicyclo[5.4.0]undec-7-en (DBU) zu. Die entstandene Lösung wird 1,5 Stunden bei Raumtemperatur gerührt und anschliessend mit 90 ml 1-n-Salzsäure sauer gestellt (pH 4). Das Acetonitril wird unter 11 Torr bei 10 °C abdestilliert. Die wässrige Suspension wird zweimal mit je 250 ml Ethylacetat extrahiert. Die organischen Phasen werden vereinigt und zweimal mit je 50 ml Wasser und zweimal mit je 50 ml Sole gewaschen, über Magnesiumsulfat getrocknet und unter 11 Torr bei 40 °C eingedampft. Der Rückstand wird an 500 g Silicagel flashchromatographiert [Eluierungsmittel:Hexan/Ethylacetat/Acetonitril (15/5/3)]. Das auf diese Weise erhaltene 2-Vinyloxybenzolsulfonsäure-N-[4-(5-cyclopentyloxycarbonylamino-1-methylindol-3-ylmethyl)-3-methoxy-benzoyl]amid wird aus Methanol umkristallisiert. Ausbeute 61 %, weisse Kristalle vom Smp. 185-188 °C.

Das Ausgangsmaterial wird wie folgt hergestellt:

Verfahrensschritt a1): 367,0 g 3-Methoxy-4-(1-methyl-5-nitroindol-3-ylmethyl)-benzoesäuremethylester werden in 3,7 l Tetrahydrofuran und 700 ml Methanol unter Erwärmen gelöst. Die dabei entstandene Lösung wird auf Raumtemperatur abgekühlt und unter Rühren mit einer Lösung von 155,0 g Lithiumhydroxid-monohydrat in 1,3 l Wasser versetzt. Die entstandene Suspension wird durch Zusatz von 1,0 l Methanol zur Lösung gebracht. Die entstandene braune, trübe Lösung wird 15 Stunden bei Raumtemperatur gerührt und nach Zusatz von Aktivkohle filtriert. Das orange Filtrat wird unter Vakuum bei 40° eingedampft. Der Rückstand wird in 2,0 l Wasser gelöst. Man versetzt die wässrige Lösung mit 350 ml 37%-iger Salzsäure und filtriert die ausgeschiedenen Kristalle ab. Den Rückstand suspendiert man in 1,5 l Wasser und filtriert

ab. Der Rückstand wird mit 500 ml Methanol gewaschen und unter 0,01 Torr bei 50° während 15 Stunden getrocknet. Die 4-(1-Methyl-5-nitroindol-3-ylmethyl)-3-methoxybenzoesäure schmilzt bei 263-265°. Ausbeute 99 %.

Verfahrensschritt a2): Eine Suspension von 340,0 g 4-(1-Methyl-5-nitroindol-3-ylmethyl)-3-methoxybenzoesäure in 12,0 l Tetrahydrofuran wird unter Rühren in einer Stickstoffatmosphäre zum Sieden erhitzt. Die dabei entstandene Lösung wird auf Raumtemperatur abgekühlt und unter Rühren mit 123,0 g N-Hydroxysuccinimid und 238,0 g N,N'-Dicyclohexylcarbodiimid versetzt. Die entstandene Suspension wird 15 Stunden bei Raumtemperatur gerührt und abfiltriert Den Rückstand (N,N'-Dicyclohexylharnstoff) wäscht man mit 200 ml Tetrahydrofuran und engt die vereinigten Filtrate unter Vakuum bei 40° zur Trockene ein. Den gelben, kristallinen Rückstand suspendiert man in 2,0 l Ethylacetat. Die Suspension wird 15 Stunden bei 5° stehengelassen und abfiltriert. Den Rückstand wäscht man mit 200 ml Ethylacetat. Der 3-Methoxy-4-(1-methyl-5-nitroindol-3-ylmethyl)-benzoesäure-N-succinimidester schmilzt bei 204-208°. Gelbe Kristalle. Ausbeute 95 %.

Verfahrensschritt a): Eine Lösung von 87,5 g 3-Methoxy-4-(1-methyl-5-nitroindol-3-ylmethyl)-benzoesäure-N-succinimidester und 70,0 g Dicyclopentyl-dicarbonat in 1,2 l Tetrahydrofuran wird nach Zusatz von 5,0 g Palladium-Kohle-Katalysator (10 %) bei Normaldruck und Raumtemperatur hydriert. Nach 4 Stunden ist die Wasserstoffaufnahme beendet. Zur Abtrennung des Katalysators wird durch eine Schicht Hyflo Super Cel filtriert und der Rückstand mit 200 ml Tetrahydrofuran nachgewaschen. Das Filtrat wird unter 11 Torr bei 40° eingedampft. Den Rückstand, ein gräulicher Kristallbrei, suspendiert man in 200 ml Diethylether. Die Suspension wird abgenutscht und der Rückstand mit 100 ml Diethylether nachgewaschen und 15 Stunden unter 0,01 Torr bei 40° getrocknet. Der 4-[5-(Cyclopentyloxycarbonylamino)-1-methylindol-3-ylmethyl]-3-methoxybenzoesäure-N-succinimidester schmilzt bei 190-193°. Ausbeute 93 %.

Herstellung des Dicyclopentyl-dicarbonats: Zu einer Lösung von 547,0 g Chlorameisensäure-cyclopentylester und 1,0 ml N,N-Dimethyloctadecylamin in 4,0 l Dichlormethan wird unter schnellem Rühren während 30 Minuten eine Lösung von 132,0 g Natriumhydroxid in 2,0 l Wasser zugetropft. Die Mischung wird anschliessend 20 Minuten bei 20-25° gerührt. Die wässrige Phase wird abgetrennt und mit 700 ml Dichlormethan extrahiert. Die vereinigten organischen Phasen werden mit 1,0 l Wasser gewaschen, über Calciumchlorid getrocknet und unter Vakuum bei 30° zur Trockene eingeengt. Der Rückstand, eine bräunliche Flüssigkeit wird in einer Kurzwegdestillationsapparatur (Graphitwischer, Turbopumpe) destilliert. Das Dicyclopentyl-dicarbonat liegt als farblose Flüssigkeit vor. Kp. 80°/0,01 Torr. Ausbeute 84 %.

Der 3-Methoxy-4-(1-methyl-5-nitroindol-3-ylmethyl)-benzoesäure-N-succinimidester [Verfahrensschritt a2)] kann auf alternative Weise wie folgt hergestellt werden:

Verfahrensschritt 1): Zu einer Lösung von 3,93 g 3-Methoxy-4-methylbenzoesäure in 100 ml Tetrahydrofuran werden unter Rühren unter Argon 2,9 g N-Hydroxysuccinimid und 5,6 g Dicyclohexylcarbodiimid zugegeben. Das Gemisch wird 18 Stunden bei Raumtemperatur gerührt. Die weisse Suspension wird abfiltriert und mit 40 ml Tetrahydrofuran nachgewaschen. Das Filtrat wird unter 11 Torr bei 40° zur Trockne eingedampft. Den Rückstand löst man in 30 ml heissem Ethylacetat. Nach Abkühlen der Lösung und Zusatz von 10 ml Ether kristallisiert der 3-Methoxy-4-methylbenzoesäure-N-succinimidester aus. Ausbeute 75,2 %, weisse Kristalle vom Smp. 134-135°.

Die Ausgangsverbindung kann z.B. wie folgt hergestellt werden:

36,0 g 3-Methoxy-4-methylbenzoesäuremethylester werden in 300 ml Methanol gelöst. Die Lösung wird mit 25 ml 30%-iger Natronlauge tropfenweise versetzt. Die Mischung wird 16 Stunden bei Raumtemperatur und 3 Stunden bei 40° gerührt und unter 11 Torr bei 50° eingedampft. Den Rückstand löst man in 500 ml Wasser und stellt die Lösung mit conc. Salzsäure sauer (pH 1). Die ausgeschiedenen weissen Kristalle werden abfiltriert mit 40 ml Wasser gewaschen und 15 Stunden unter 0,1 Torr bei 90° getrocknet. Die 3-Methoxy-4-methylbenzoesäure schmilzt bei 157-158°. Ausbeute 96 %.

Verfahrensschritt 2): Eine Suspension von 14,05 g 3-Methoxy-4-methylbenzoesäure-N-succinimidester in 160 ml Tetrachlormethan wird unter Rühren bei Raumtemperatur unter Stickstoff mit 10,95 g N-Bromsuccinimid und 0,5 g Azoisobutyronitril (AIBN) versetzt. Die Mischung wird 24 Stunden unter Rückfluss erhitzt, abgekühlt auf Raumtemperatur und abfiltriert. Das Filtrat wird unter 11 Torr bei 50° eingedampft. Den Rückstand kristallisiert man aus einem Gemisch von 100 ml Ethylacetat und 200 ml Hexan. Der 4-Brommethyl-3-methoxy-benzoesäure-N-succinimidester schmilzt bei 124-125°. Beige Kristalle. Ausbeute 74,8 %.

Verfahrensschritt 3): 2,85 g N-Methyl-5-nitroindol und 7,5 g Silbercarbonat werden unter Rühren in einer Argonatmosphäre in 100 ml Toluol suspendiert. Die Suspension wird 18 Stunden unter Rückfluss erhitzt, auf 55° abgekühlt und unter Rühren tropfenweise mit einer Lösung von 5,4 g 4-Brommethyl-3-methoxy-benzoesäure-N-succinimidester versetzt. Die Mischung wird fünf Tage bei 55-60° gerührt, auf Raumtemperatur abgekühlt und abfiltriert. Der Rückstand wird mit 30 ml Toluol nachgewaschen. Das Filtrat wird unter

11 Torr bei 50° eingedampft. Der Rückstand wird an 1000 g Silicagel über eine MPLC-Kolonne chromatographiert (Eluierungsmittel Ethylacetat/Hexan 3:2). Der auf diese Weise erhaltene 3-Methoxy-4-(1-methyl-5-nitroindol-3-ylmethyl)-benzoesäure-N-succinimidester wird aus Ethylacetat umkristallisiert. Ausbeute 37 %, gelbe Kristalle vom Smp. 215-216°.

Beispiel 2: [Verfahrensschritt b)] Analog Beispiel 1 erhält man das 2-Vinyloxybenzolsulfonsäure-N-[4-(5-cyclopentyloxycarbonylamino-1-methylindol-3-ylmethyl)-3-methoxy-benzoyl]amid ausgehend von 4-[5-(Cyclopentyloxycarbonylamino)-1-methylindol-3-ylmethyl]-3-methoxy-benzoesäure-2,4,5-trichlorphenylester und 2-Vinyloxybenzolsulfonsäureamid mit einer Ausbeute von 62 %.

Das Ausgangsmaterial wird wie folgt hergestellt:

Verfahrensschritt a2): Eine Mischung von 2,38 g 4-(1-Methyl-5-nitroindol-3-ylmethyl)-3-methoxybenzoesäure [Beispiel 1, Verfahrensschritt a1)], 1,48 g 2,4,5-Trichlorphenol und 1,0 g 4-Dimethylaminopyridin in 140 ml Tetrahydrofuran wird unter Rühren in einer Stickstoffatmosphäre bei Raumtemperatur mit 1,66 g Dicyclohexylcarbodiimid versetzt. Das Reaktionsgemisch wird 15 Stunden bei Raumtemperatur gerührt und filtriert. Das Filtrat wird unter Vakuum bei 50° eingedampft. Der Rückstand wird in 400 ml Chloroform unter Erwärmen gelöst. Man wäscht die organische Phase bei Raumtemperatur mit 50 ml 2-n. Salzsäure und 50 ml Wasser, trocknet sie über Magnesiumsulfat und engt die Lösung unter 11 Torr bei 40° zur Trockene ein. Der Rückstand wird an 350 g Silicagel chromatographiert) Eluierungsmittel Dichlormethan). Der auf diese Weise erhaltene 3-Methoxy-4-(1-methyl-5-nitroindol-3-ylmethyl)-benzoesäure-2,4,5-trichlorphenylester wird mit 50 ml Ether aufgeschlämmt und abfiltriert. Ausbeute 67 %. Gelbe Kristalle vom Smp. 235-236°.

Verfahrensschritt a): Eine Suspension von 1,04 g 3-Methoxy-4-(1-methyl-5-nitroindol-3-yl-methyl)-benzoesäure-2,4,5-trichlorphenylester, 0,6 g Dicyclopentyl-dicarbonat und 1,0 g 1,2-Dichlorbenzol in 60 ml Tetrahydrofuran wird nach Zusatz von 0,4 g Palladium-Kohle-Katalysator (10 %) 25 Stunden bei Raumtemperatur hydriert. Es werden nochmals 0,8 g Katalysator und 0,5 g 1,2-Dichlorbenzol zugesetzt und weitere 45 Stunden hydriert. Zur Abtrennung des Katalysators wird die Mischung filtriert und der Rückstand mit 50 ml Tetrahydrofuran nachgewaschen. Das Filtrat wird unter Vakuum zur Trockene eingedampft. Der Rückstand wird mit 100 ml Petrolether verrieben, abfiltriert und an 190 g Silicagel über eine MPLC-Kolonne chromatographiert (Eluierungsmittel Dichlormethan). Der so erhaltene 4-[5-(Cyclopentyloxycarbonylamino)-1-methylindol-3-ylmethyl]-3-methoxy-benzoesäure-2,4,5-trichlorphenylester wird aus Ether-Petrolether umkristallisiert. Ausbeute 35 %. Farblose Kristalle vom Smp. 134-136°.

## Patentansprüche

1. Verfahren zur Herstellung einer Verbindung der Formel I,

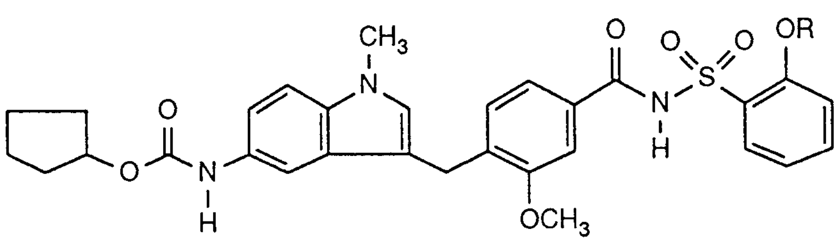

(I)

worin R geradkettiges $C_2$-$C_4$-Alk-1-en-1-yl bedeutet, oder einem Salz davon, dadurch gekennzeichnet, dass man

a) eine Verbindung der Formel IV,

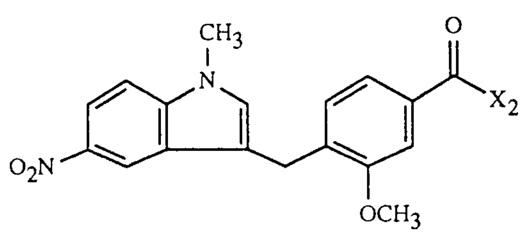

(IV)

worin $X_2$ für

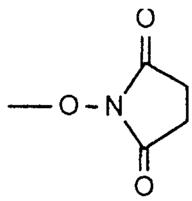 (IVa), 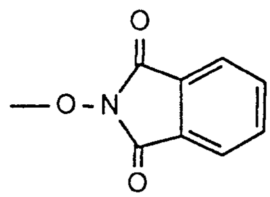 (IVb) oder

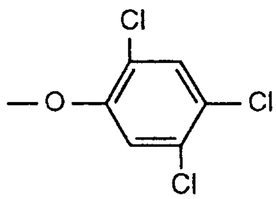 (IVc)

steht, in einem Eintopfverfahren, d.h. ohne Isolierung von Zwischenprodukten, reduziert und gleichzeitig mit der Verbindung der Formel V

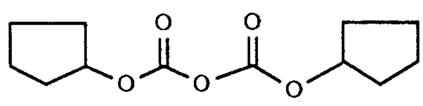 (V)

N-acyliert; und

b) eine so erhältliche Verbindung der Formel VIa

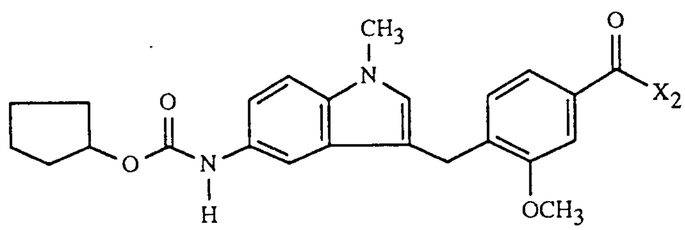 (VIa)

mit einer Verbindung der Formel VIb,

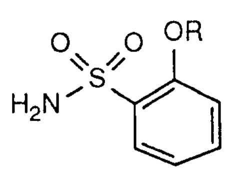 (VIb)

oder einem Salz davon, umsetzt.

2. Verfahren gemäss Anspruch 1, dadurch gekennzeichnet, dass man zur Herstellung der in Anspruch 1 als Ausgangsmaterial verwendeten Verbindung der Formel IV

a1) einen Ester der Formel II,

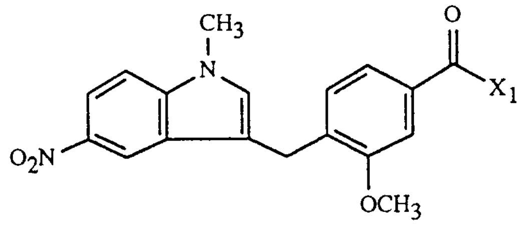
(II)

worin $X_1$ verethertes Hydroxy bedeutet, hydrolysiert; und
a2) in der so erhältlichen Carbonsäure der Formel III

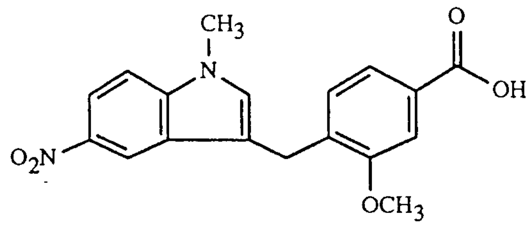
(III)

die Carboxygruppe durch Umsetzung mit einer Verbindung der Formel

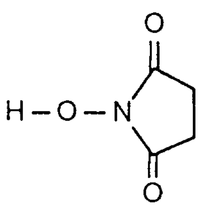 (IVd), 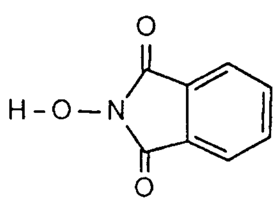 (IVe) oder 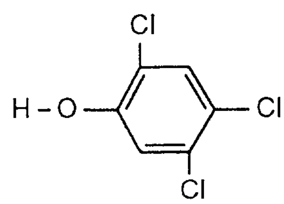 (IVf)

aktiviert.

**3.** Verfahren gemäss Anspruch 1, dadurch gekennzeichnet, dass man zur Herstellung der in Anspruch 1 als Ausgangsmaterial verwendeten Verbindung der Formel IV
1) in der Verbindung der Formel IIIa

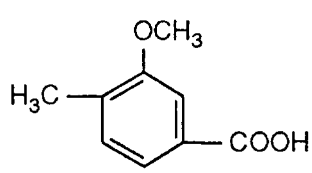
(IIIa)

die Carboxygruppe durch Umsetzung mit einer Verbindung der Formel

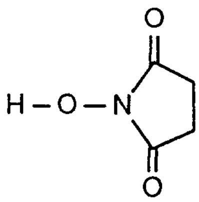 (IVd), 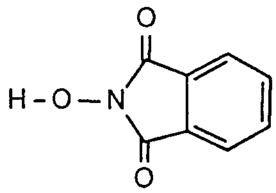 (IVe) oder 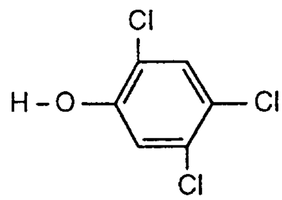 (IVf)

aktiviert; und

2) eine so erhältliche Verbindung der Formel IIIb,

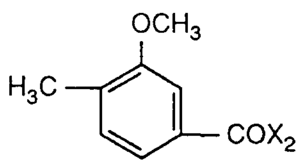 (IIIb)

worin $X_2$ für

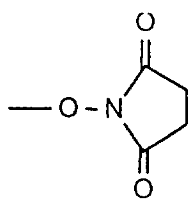 (IVa), 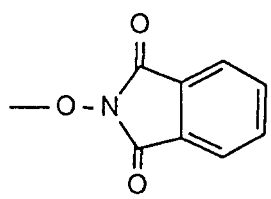 (IVb) oder

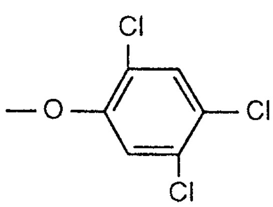 (IVc)

steht, halogeniert; und

3) eine so erhältliche Verbindung der Formel IIIc,

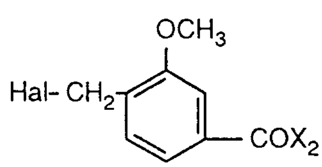 (IIIc)

worin Hal Halogen bedeutet und $X_2$ wie unter Formel IIIb definiert ist, mit der Verbindung der Formel IIId

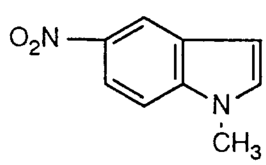 (IIId)

umsetzt.

4. Verfahren gemäss einem der Ansprüche 1 bis 3, dadurch gekennzeichnet, dass man die N-Acylierung einer Verbindung der Formel VIb mit der Verbindung der Formel VIa [Verfahrensschritt b)] in Gegenwart einer Base ausgewählt aus 1,8-Diazabicyclo[5.4.0]undec-7-en, 1,5-Diazabicyclo[4.3.0]non-5-en, einem Alkalimetallalkoholat und Benzylthmethylammoniumhydroxid durchführt.

5. Verfahren gemäss Anspruch 4, dadurch gekennzeichnet, dass man die N-Acylierung einer Verbindung der Formel VIb mit der Verbindung der Formel VIa [Verfahrensschritt b)] in Gegenwart von 1,8-Diazabicyclo[5.4.0]undec-7-en durchführt.

6. Verfahren gemäss einem der Ansprüche 1-5, dadurch gekennzeichnet, dass die Verbindung der Formel I, worin R Vinyl bedeutet, oder ein Salz davon, hergestellt wird.

7. Dicyclopentyl-dicarbonat.

8. Verwendung von Dicyclopentyl-dicarbonat als Acylierungsmittel bei der Reduktion und N-Acylierung von Nitroindolderivaten der Formel IV,

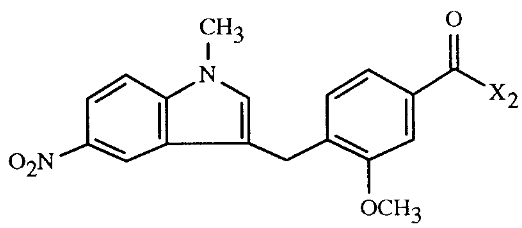

(IV)

worin $X_2$ für

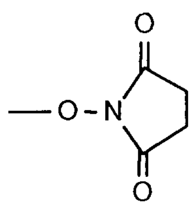 (IVa), 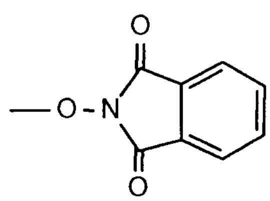 (IVb) oder

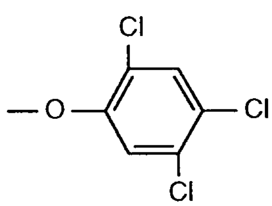 (IVc)

steht,
im Eintopfverfahren.

## Claims

**1.** A process for the preparation of a compound of formula I

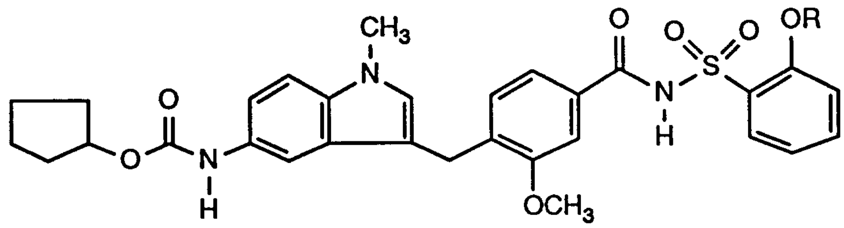

(I)

wherein R is straight-chain $C_2$-$C_4$ alk-1-en-1-yl, or a salt thereof, wherein

a) a compound of formula IV

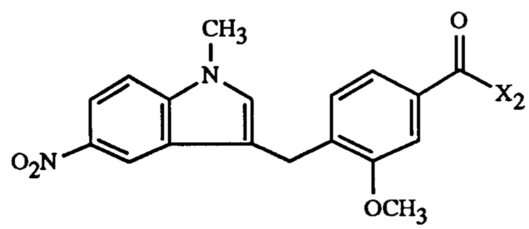

(IV),

wherein $X_2$ is

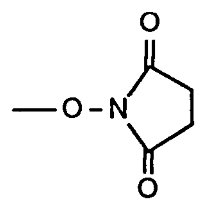

(IVa),

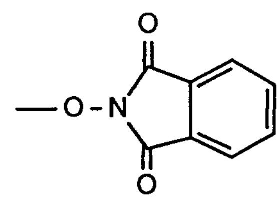

(IVb) or

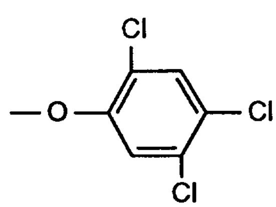

(IVc),

is reduced in a one-pot process, that is to say without isolation of intermediates, and is simultaneously N-acylated with the compound of formula V

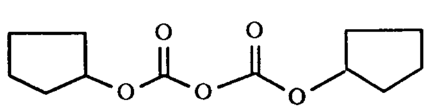

(V);

and

b) a compound of formula VIa so obtainable

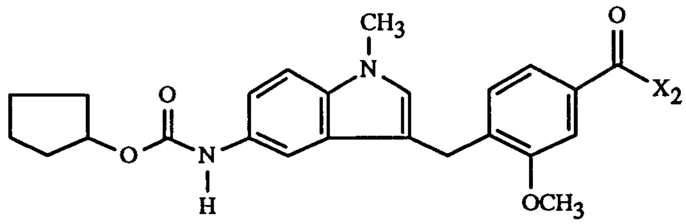

(VIa)

is reacted with a compound of formula VIb

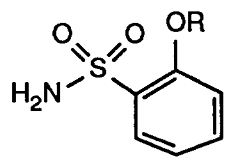

(VIb)

or with a salt thereof.

**2.** A process according to claim 1, wherein, for the preparation of the compound of formula IV used as starting material in claim 1,

a1) an ester of formula II

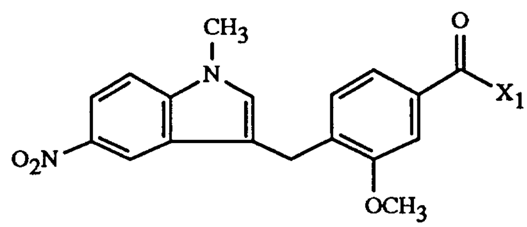

(II)

wherein $X_1$ is etherified hydroxy, is hydrolysed; and

a2) in the carboxylic acid of formula III so obtainable

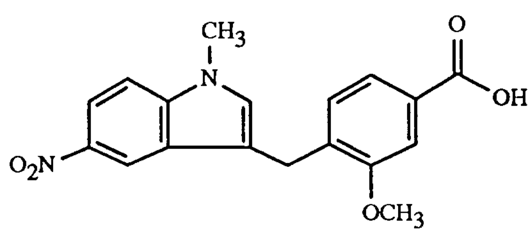

(III)

the carboxy group is activated by reaction with a compound of the formula

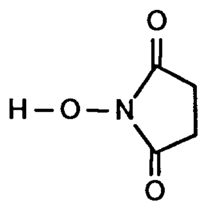 (IVd), 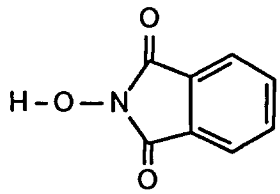 (IVe) or 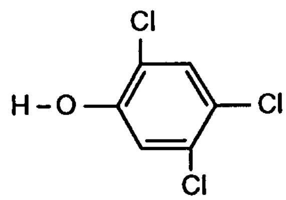 (IVf).

3. A process according to claim 1, wherein, for the preparation of the compound of formula IV used as starting material in claim 1,

1) in the compound of formula IIIa

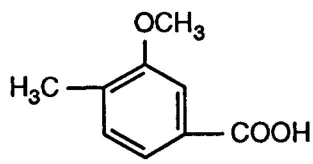

(IIIa)

the carboxy group is activated by reaction with a compound of the formula

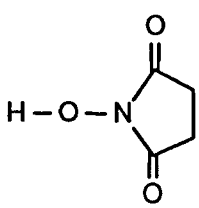 (IVd), 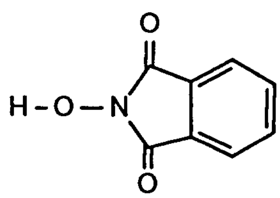 (IVe) or 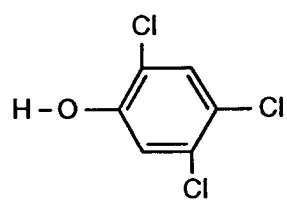 (IVf) ;

and

2) a compound of formula IIIb so obtainable

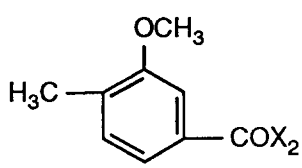

(IIIb),

wherein $X_2$ is

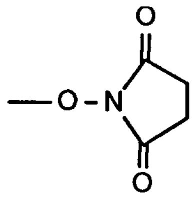 (IVa), 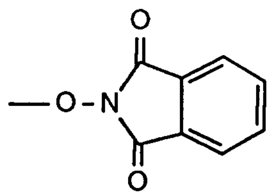 (IVb) or

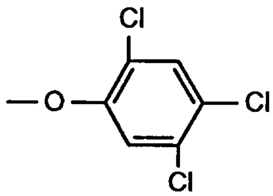 (IVc),

is halogenated; and

3) a compound of formula IIIc so obtainable

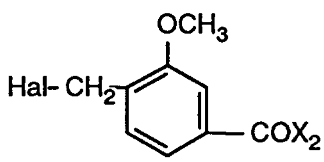 (IIIc),

wherein Hal is halogen and $X_2$ is as defined under formula IIIb, is reacted with the compound of formula IIId

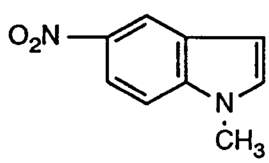 (IIId).

**4.** A process according to any one of claims 1 to 3, wherein the N-acylation of a compound of formula VIb with a compound of formula VIa [Process Step b)] is carried out in the presence of a base selected from 1,8-diazabicyclo[5.4.0]undec-7-ene, 1,5-diazabicyclo[4.3.0]non-5-ene, an alkali metal alcoholate and benzyltrimethylammonium hydroxide.

**5.** A process according to claim 4, wherein the N-acylation of a compound of formula VIb with a compound of formula VIa [Process Step b)] is carried out in the presence of 1,8-diazabicyclo[5.4.0]-undec-7-ene.

**6.** A process according to any one of claims 1 to 5, wherein the compound of formula I wherein R is vinyl, or a salt thereof, is prepared.

**7.** Dicyclopentyl dicarbonate.

**8.** The use of dicyclopentyl dicarbonate as an acylation agent in the reduction and N-acylation of nitroindole derivatives of formula IV

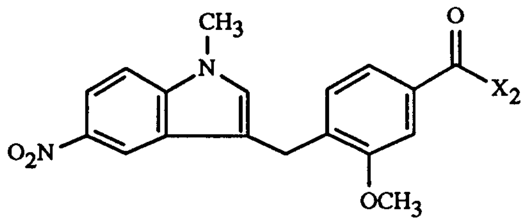 (IV),

wherein $X_2$ is

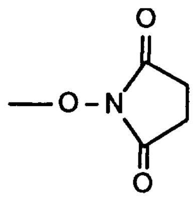 (IVa), 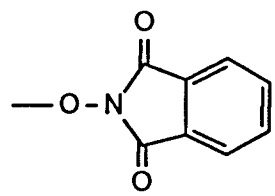 (IVb) or

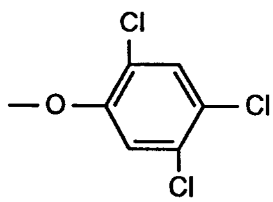 (IVc),

in a one-pot process.

**Revendications**

**1.** Procédé de préparation d'un composé de formule I

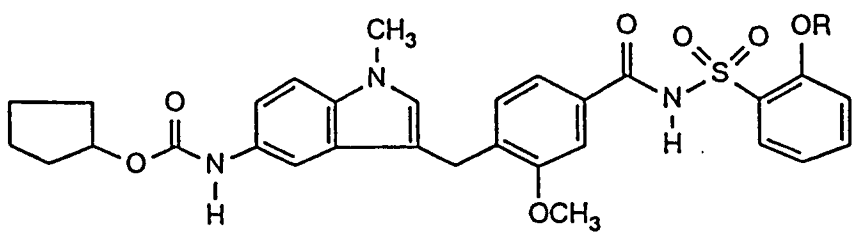 (I)

dans laquelle R représente un groupe alcène-1-yle à chaîne droite, ou d'un sel d'un tel composé, caractérisé en ce que

a) on réduit un composé de formule IV

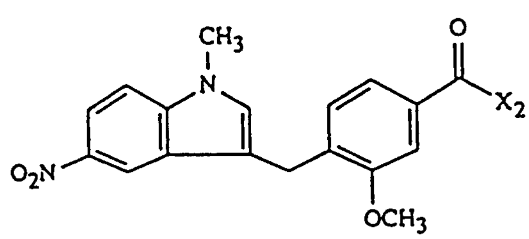 (IV)

dans laquelle $X_2$ représente

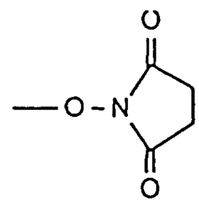
(IVa),

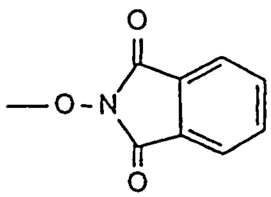
(IVb) ou 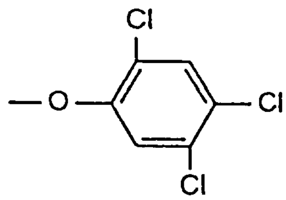
(IVc)

dans une opération en un seul récipient, c'est-à-dire sans isoler les produits intermédiaires, et on le soumet simultanément à N-acylation à l'aide du composé de formule V

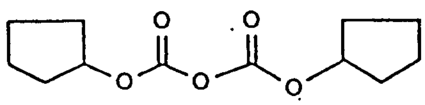
(V)

et

b) on fait réagir le composé ainsi obtenu répondant à la formule VIa

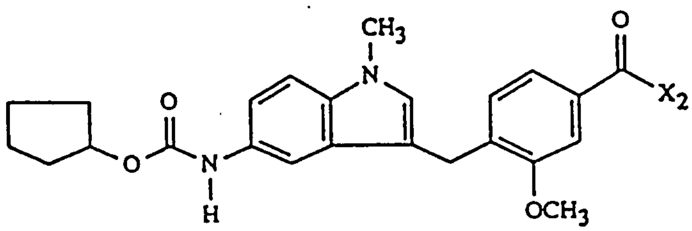
(VIa)

avec un composé de formule VIb

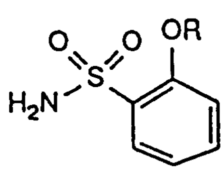
(VIb)

ou l'un de ses sels.

**2.** Procédé selon revendication 1, caractérisé en ce que pour la préparation du composé de formule IV utilisé en tant que produit de départ selon revendication 1,

a1) on hydrolyse un ester de formule II

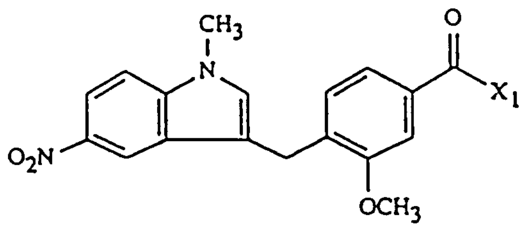

(II)

dans laquelle $X_1$ représente un groupe hydroxy éthérifié ; et

a2) dans l'acide carboxylique ainsi obtenu, répondant à la formule III

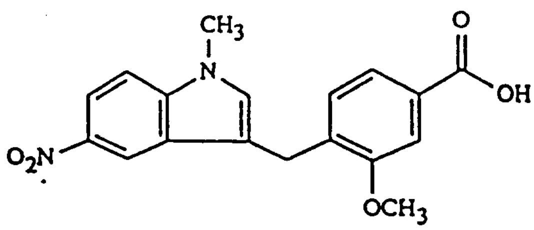

(III)

on active le groupe carboxy par réaction avec un composé de formule

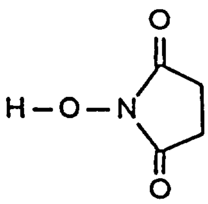 (IVd), 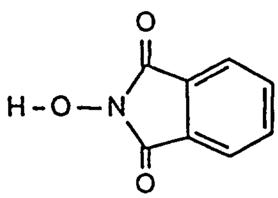 (IVe) ou 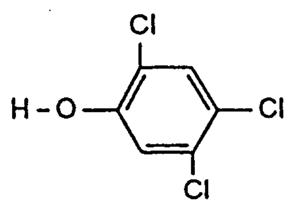 (IVf)

**3.** Procédé selon revendication 1, caractérisé en ce que, pour la préparation du composé de formule IV utilisé en tant que produit de départ selon revendication 1,

1) dans le composé de formule IIIa

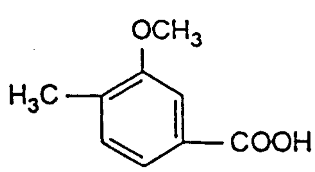

(IIIa)

on active le groupe carboxy par réaction avec un composé de formule

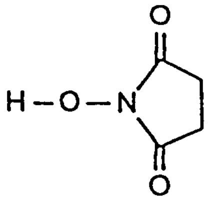 (IVd), 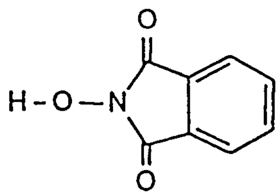 (IVe) ou 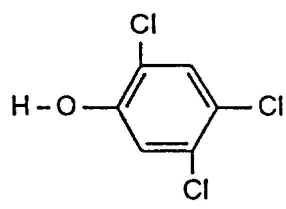 (IVf)

et

2) on halogène le composé ainsi obtenu, répondant à la formule IIIb

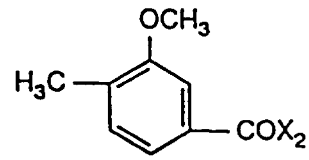

(IIIb)

dans laquelle $X_2$ représente

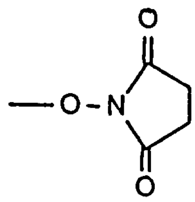

(IVa),

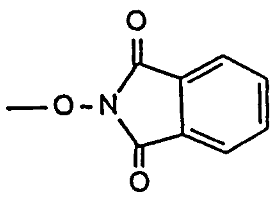

(IVb) ou

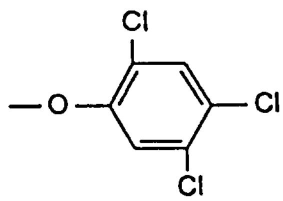

(IVc)

et

3) on fait réagir le composé ainsi obtenu, répondant à la formule IIIc

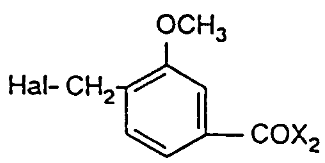

(IIIc)

dans laquelle Hal représente un halogène et $X_2$ a les significations indiquées en référence à la formule IIIb, avec le composé de formule IIId

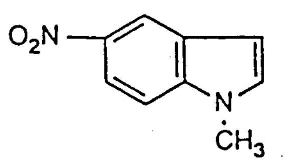

(IIId)

**4.** Procédé selon l'une des revendications 1 à 3, caractérisé en ce que la N-acylation d'un composé de formule VIb) à l'aide du composé de formule VIa) (stade opératoire b)) est effectuée en présence d'une base choisie parmi le 1,8-diazabicyclo[5.4.0]-undéca-7-ène, le 1,5-diazabicyclo[4.3.0]nona-5-ène, un alcoolate de métal alcalin et l'hydroxyde de benzyltriméthylammonium.

**5.** Procédé selon revendication 4, caractérisé en ce que la N-acylation d'un composé de formule VIb par le composé de formule VIa (stade opératoire b)) est effectuée en présence du 1,8-diazabicyclo[5.4.0]-undéca-7-ène.

**6.** Procédé selon l'une des revendications 1 à 5, caractérisé en ce que l'on prépare le composé de formule I dans laquelle R représente un groupe vinyle ou l'un de ses sels.

7. Le dicarbonate de dicyclopentyle.

8. L'utilisation du dicarbonate de dicyclopentyle en tant qu'agent acylant à la réduction et à la N-acylation de dérivés du nitroindole répondant à la formule IV

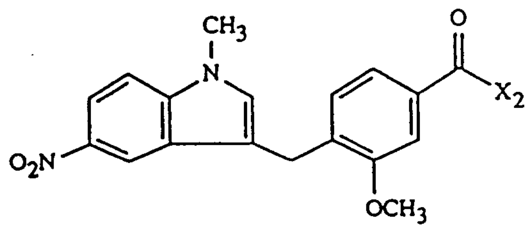

(IV)

dans laquelle $X_2$ représente

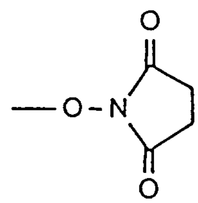

(IVa),

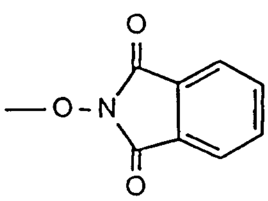

(IVb) ou

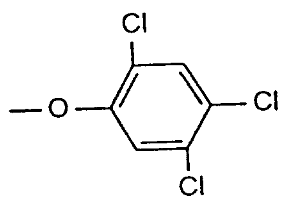

(IVc)

dans des opérations en un seul récipient.